# EUROPEAN PATENT APPLICATION

(11) **EP 2 087 896 A1**
(43) Date of publication of application: **12.08.2009**
(21) Application number: 09151449.7
(22) Date of filing: 27.01.2009
(51) Int. Cl.: A61K 35/20, A61K 31/4415, A61K 36/738, A61P 1/02

(54) **Topical compositions comprising colostrum, folates and honey of rose for the treatment of disorders of the oral cavity**

(30) Priority: 05.02.2008 IT MI20080174
(71) Applicant: YOU Biomedical Research S.r.l., 25100 Brescia (IT)
(72) Inventor: Gobbi, Maria Rosa, 22073, FINO MORNASCO (CO) (IT)
(74) Representative: Minoja, Fabrizio

(57) **Abstract**

This invention relates to topical compositions comprising colostrum, folates and honey of rose for the treatment of disorders of the oral cavity.

## Description

### FIELD OF INVENTION

The present invention relates to topical compositions comprising colostrum, folates and honey of rose for the treatment of disorders of the oral cavity.

### BACKGROUND OF THE INVENTION

Disorders of the oral cavity, especially inflammatory disorders such as stomatitis, gingivitis and the like, may be caused by one or more factors, such as poor oral hygiene, infectious agents, avitaminosis, local traumas and autoimmune diseases. Consequently, the treatment may simply require better oral hygiene or involve the elimination of mechanical factors such as malocclusion, ill-fitting prostheses, etc. or the use of antibacterial, astringent and anti-inflammatory agents, antibiotics, antifungals and local anaesthetics. However, pharmacological treatment often has undesirable side effects, or can be limited or precluded by the patient's age or state of health. A further problem is that the contact time between the oral mucosa and the active agent is generally short.

A particularly important aspect of oral cavity disorders is the eruption of the milk teeth in children, which begins at the age of 4-6 months and is complete by about two years old. This teething process is often accompanied by disorders ranging from a mere sensation of discomfort or itching of the gingival mucous membranes, which causes the child to try and bite everything within reach (hard objects, edges of the pushchair, parents' fingers, etc.) to painful sensations caused by inflammation of the gum, which swells as it is pushed by the tooth about to erupt. The child's pain can be severe enough to cause irritability, loss of appetite and great agitation, especially during the night. Teething problems can be dealt with by giving the child specially designed non-toxic rubber toys to chew so that they massage the gums or, if the child manifests greater discomfort or pain, using topical formulations with an anaesthetic action, comprising amylocaine, lidocaine, benzocaine or the like, which are spread on the child's gums to alleviate the pain.

The need is strongly felt for a topical formulation which, while performing a significant anti-inflammatory, antibacterial and analgesic action in the oral cavity, does not cause undesirable side effects, and remains in contact with the oral mucosa for a sufficient time.

When administered to patients suffering from disorders of the oral cavity, colostrum has an anti-inflammatory effect and rapidly restores the physiological conditions normally present in the buccal mucosa. Moreover, colostrum has an excellent ability to adhere to the oral mucosa, thus giving the formulations according to the invention contact times which are effective in the treatment of disorders of the oral cavity.

Honey of rose is a preparation widely used in traditional medicine to treat forms of gingival inflammation, especially during teething. Honey of rose is prepared by mixing honey with rose petal extract (rich in tannins, anthocyanic heterosides and flavone derivatives), which has a mild astringent and decongestant action.

Folates are used (in the form of calcium folinate) to treat anaemia caused by folic acid deficiencies resulting from increased demand, insufficient dietary intake or reduced use, to treat methotrexate overdose, and to prevent and reduce the toxic effects caused by treatment with high doses of methotrexate.

### DESCRIPTION OF THE INVENTION

It has now been found that a combination of colostrum, folates and honey of rose, when administered to patients suffering from inflammatory disorders of the oral cavity, performs an anti-inflammatory action, restoring the physiological conditions of the mucous membranes of the oral cavity. The mild anaesthetic action of honey of rose and of colostrums also contributes to the patient's well-being.

The present invention therefore relates to topical compositions containing:
a) colostrum
b) folates and
c) honey of rose
for the treatment of disorders of the oral cavity.

The compositions according to the invention will preferably contain:
a) 2 to 40% colostrum;
b) 0.001 to 0.002% folates (0.0017%);
c) 5 to 50% honey of rose (0.08%).

According to a particularly preferred aspect, the compositions according to the invention will contain:
a) 5 to 20% colostrum;
b) 0.001 to 0.002% folates;
c) 10 to 50% honey of rose.

According to the invention, the animal colostrum may be obtained from any type of mammal, but will preferably be equine or bovine.

According to a preferred aspect of invention, the colostrum used will be that taken from the animal within the first hour of production (H1 colostrum).

According to the invention, the colostrum may be present in the compositions in powder or liquid form; in particular, powdered colostrum may be in freeze-dried or spray-dried form.

The effect of the compositions according to the invention is greater than the sum of the effects obtained by separate administration of the individual constituents of the combination. This superior effect is apparently due to synergy between the various constituents.

The compositions according to the invention will be presented in the form of a gingival paste or gel to be rubbed into the gums, liquid mouthwashes or mouthwashes in sachets to be diluted at the time of use, for application to the oral cavity by gargling or rinsing with a neat solution, non-gas sprays, or toothpastes.

The compositions according to the invention will be used by the patient 1 to 3 times a day, especially morning and night after cleaning the oral mucous membranes, or at other appropriate times.

According to a further aspect thereof, the compositions according to the invention will also contain antibacterial or anti-inflammatory agents, local anaesthetics, plant extracts with an astringent, anti-inflammatory or anaesthetic action, and compounds with an adjuvant and/or complementary action in general.

The compositions according to the invention will be formulated according to conventional pharmaceutical techniques to prepare topical formulations for the treatment of the oral cavity, on the basis of conventional methods well known in pharmaceutical technology, such as those described in "Remington's Pharmaceutical Handbook", Mack Publishing Co., N.Y., USA, using excipients, diluents, filling agents and anti-caking agents acceptable for their final use.

The following is an example of a formulation according to the invention.

### EXAMPLE

| | |
|---|---|
| Colostrum | 10% |
| Folates | 0.0017% |
| Honey of rose | 30% |
| Sweetener: xylitol | 5% |
| Preservatives: | |
| sodium benzoate | 0.1 % |
| potassium sorbate | 0.1 % |
| Rheology modifiers: | |
| carrageenans | 0.1% |
| sodium carboxymethyl-cellulose | 0.1 % |
| Flavouring agents: natural orange blossom flavouring | 0.03% |
| Purified water: q.s. for 100. | |

## Claims

1. Topical oral compositions containing:
a) colostrum
b) folates and
c) honey of rose.

2. Compositions as claimed in claim 1, containing:
a) 2 to 40% colostrum, preferably bovine;
b) 0.001 to 0.002% folates (0.0017%);
c) 5 to 50% honey of rose (0.08%).

3. Compositions as claimed in claim 2, containing:
a) 5 to 20% colostrum;
b) 0.001 to 0.002% folates;
c) 10 to 50% honey of rose.

4. Compositions as claimed in the preceding claims containing colostrum, preferably bovine.

5. Compositions as claimed in the preceding claims, containing colostrum in powder or liquid form.

6. Compositions as claimed in claim 5, wherein the powdered colostrum is in freeze-dried or spray-dried form.

7. Compositions as claimed in the preceding claims, in the form of gingival pastes or gels, liquid mouthwashes or mouthwashes in sachets to be diluted at the time of use, or toothpastes.

8. The use of:
a) colostrums
b) folates and
c) honey of rose
for the preparation of compositions for the treatment of disorders of the oral cavity.
